(19) Europäisches Patentamt
European Patent Office
Office européen des brevets

(11) **EP 2 834 625 B1**

(12) **FASCICULE DE BREVET EUROPEEN**

(45) Date de publication et mention de la délivrance du brevet:
**24.10.2018 Bulletin 2018/43**

(51) Int Cl.:
***G01N 25/00*** *(2006.01)*   ***G01R 31/28*** *(2006.01)*
***H01L 21/66*** *(2006.01)*

(21) Numéro de dépôt: **13722478.8**

(22) Date de dépôt: **05.04.2013**

(86) Numéro de dépôt international:
**PCT/FR2013/000090**

(87) Numéro de publication internationale:
**WO 2013/150194 (10.10.2013 Gazette 2013/41)**

(54) **DETERMINATION DE LA CONCENTRATION EN OXYGENE INTERSTITIEL DANS UN ECHANTILLON SEMI-CONDUCTEUR**

BESTIMMUNG DER KONZENTRATION VON INTERSTITIELLEM SAUERSTOFF IN EINER HALBLEITERPROBE

DETERMINATION OF THE CONCENTRATION OF INTERSTITIAL OXYGEN IN A SEMICONDUCTOR SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.04.2012 FR 1201045**

(43) Date de publication de la demande:
**11.02.2015 Bulletin 2015/07**

(73) Titulaire: **Commissariat à l'Énergie Atomique et aux Énergies Alternatives 75015 Paris (FR)**

(72) Inventeurs:
 • **VEIRMAN Jordi**
   **FR-74330 Poisy (FR)**
 • **DUBOIS Sébastien**
   **FR-74950 Scionzier (FR)**
 • **ENJALBERT Nicolas**
   **FR-81 100 Burlats (FR)**

(74) Mandataire: **Talbot, Alexandre Cabinet Hecké 28 Cours Jean Jaurès 38000 Grenoble (FR)**

(56) Documents cités:
   **EP-A1- 2 697 632**

 • SIMOEN E ET AL: "Characterisation of oxygen and oxygen-related defects in highly- and lowly-doped silicon", MATERIALS SCIENCE AND ENGINEERING B, ELSEVIER SEQUOIA, LAUSANNE, CH, vol. 102, no. 1-3, 15 septembre 2003 (2003-09-15), pages 207-212, XP004450731, ISSN: 0921-5107, DOI: 10.1016/S0921-5107(02)00706-7
 • ULYASHIN A G ET AL: "Characterization of the oxygen distribution in Czochralski silicon using hydrogen-enhanced thermal donor formation", MATERIALS SCIENCE AND ENGINEERING B, ELSEVIER SEQUOIA, LAUSANNE, CH, vol. 73, no. 1-3, 1 avril 2000 (2000-04-01), pages 124-129, XP004192033, ISSN: 0921-5107, DOI: 10.1016/S0921-5107(99)00447-X cité dans la demande
 • SASSELLA A ET AL: "Influence of oxygen precipitation on the measure of interstitial oxygen concentration in silicon from the 1207 cm-1 infrared absorption band", JOURNAL OF APPLIED PHYSICS, AMERICAN INSTITUTE OF PHYSICS. NEW YORK, US, vol. 91, no. 1, 1 janvier 2002 (2002-01-01), pages 166-170, XP012054470, ISSN: 0021-8979, DOI: 10.1063/1.1423393

- **SAITO H ET AL: "Determination of Interstitial Oxgen Concentration in Oxygen-Precipitated Silicon Wafers by Low-Temperature High-Resolution Infrared Spectroscopy", JAPANESE JOURNAL OF APPLIED PHYSICS, THE JAPAN SOCIETY OF APPLIED PHYSICS, JAPAN SOCIETY OF APPLIED PHYSICS, TOKYO; JP, vol. 34, no. 9A, PART 02, 1 septembre 1995 (1995-09-01), pages L1097-L1099, XP002115943, ISSN: 0021-4922, DOI: 10.1143/JJAP.34.L1097**

**Description**

**Domaine technique de l'invention**

L'invention est relative à un procédé permettant de déterminer la concentration en oxygène interstitiel d'un échantillon semi-conducteur de type p.

**État de la technique**

Les substrats en silicium destinés à l'industrie microélectronique ou aux applications photovoltaïques comprennent de l'oxygène. Lorsqu'ils ne sont pas sous forme de précipités, les atomes d'oxygène occupent généralement des positions interstitielles dans le réseau cristallin. Dans le cas du silicium monocristallin, obtenu par le procédé Czochralski, ou dans le cas du silicium polycristallin de qualité dite « solaire », la concentration en oxygène interstitiel $C_O$ varie entre $10^{17}$ et $2.10^{18}$ atomes/cm$^3$.

L'oxygène en position interstitielle ($O_i$) joue un rôle important sur les propriétés mécaniques et électriques du silicium. En particulier, à des températures comprises entre 200 °C et 500 °C, l'oxygène forme des agglomérats appelés Doubles Donneurs Thermiques (DDT) qui modifient les propriétés électriques du matériau. A plus haute température, l'oxygène forme des précipités permettant de piéger des impuretés métalliques présentes dans le silicium. Un effet getter peut ainsi être obtenu. Par ailleurs, l'oxygène améliore les propriétés mécaniques des substrats en bloquant les dislocations introduites par les procédés de fabrication.

Pour les applications photovoltaïques, une concentration en oxygène élevée entraîne une diminution des performances sous éclairement, notamment une diminution du rendement de conversion des cellules photovoltaïques à base de silicium dopé p (dont les porteurs majoritaires sont des trous). En particulier, c'est le cas des cellules à base de silicium dopé au bore (B).

Il paraît donc important de connaître la concentration et la répartition de l'oxygène interstitiel au sein d'un substrat de type p pour déterminer localement l'influence de l'oxygène sur les propriétés électriques et mécaniques du silicium. Ces informations permettent ensuite d'optimiser les procédés de cristallisation ou de fabrication des dispositifs.

La concentration en oxygène d'un échantillon semi-conducteur est classiquement déterminée par spectroscopie infrarouge FTIR (« Fourier Transform InfraRed spectroscopy »). Cependant, cette technique est lente et imprécise. Par ailleurs, elle nécessite un échantillon d'au moins 200 $\mu$m d'épaisseur et une préparation de la surface de l'échantillon.

En outre, la spectroscopie infrarouge FTIR ne permet pas de mesurer précisément des concentrations $C_O$ inférieures à $10^{16}$ cm$^{-3}$. D'ailleurs, cette concentration limite est encore plus élevée dans un silicium fortement dopé, où le dopage net $N_A$-$N_D$ est typiquement supérieur à $5.10^{16}$ cm$^{-3}$. En effet, l'absorption par les nombreux porteurs de charge perturbe la mesure.

L'article « Characterization of the oxygen distribution in Czochralski silicon using hydrogen-enhanced thermal donor formation » (A.G. Ulyashin et al., Materials Science and Engineering B73, 124-129, 2000) décrit une autre technique de détermination de la concentration en oxygène.

Cette technique est basée sur la formation de donneurs thermiques DDT. Un traitement thermique assisté par un plasma hydrogène est appliqué à un échantillon en silicium de type p de manière à former une jonction p-n. Puis, la profondeur de la jonction p-n dans l'échantillon est déterminée à l'aide de mesures de résistance de type SRP (« Spreading Résistance Probe » en anglais) ou de mesures de capacité C-V (« Capacitance-Voltage » en anglais). La concentration en donneurs thermiques est ensuite calculée à partir de la profondeur de la jonction p-n, ou est tirée de la concentration en électrons générées par ces donneurs. Un modèle mathématique permet de déterminer la concentration en oxygène à partir de la concentration en donneurs thermiques.

Les méthodes de caractérisation employées requièrent, au même titre que la FTIR, une préparation de l'échantillon. La caractérisation SRP nécessite de biseauter l'échantillon pour établir le profil de résistance sur la profondeur de l'échantillon. La caractérisation C-V utilise des contacts métalliques à la surface de l'échantillon. Ces contacts sont difficiles à enlever sans dégrader ou polluer le matériau de l'échantillon.

En raison de la complexité de ces méthodes de caractérisation, la technique de mesure de l'article susmentionné est lente et difficilement applicable à des substrats de l'industrie microélectronique et photovoltaïque. De plus, la préparation et l'hydrogénation du substrat rendent celui-ci inutilisable à l'issue de la mesure.

Par ailleurs, cette technique ne s'applique pas à des substrats fortement dopés. Pour ces substrats, la quantité de donneurs thermiques formés n'est pas suffisante pour faire apparaître la jonction p-n nécessaire à la mesure.

L'article « Characterisation of oxygen and oxygen-related defects in highly-and lowly-doped silicon » (E. Simoen et al., Materials Science and Engineering B102, 207-212, 2003) décrit les mêmes techniques pour étudier l'oxygène et les précipités d'oxygène dans du silicium faiblement dopé à fortement dopé. En particulier, la spectroscopie infrarouge FTIR, à basse température, permet de mesurer la concentration en oxygène interstitiel dans un silicium de type p fortement dopé. Les techniques de caractérisation SRP et C-V mettent en évidence la présence de donneurs thermiques dans du silicium de haute résistivité dopé à l'oxygène, ayant subi un recuit thermique à 450 °C.

La demande FR2964459 décrit une technique permettant de cartographier la concentration en oxygène d'un substrat en silicium. Cette technique repose également sur la formation de donneurs thermiques DDT dans le silicium. Une mesure de résistivité est réalisée avant et après un recuit d'activation des donneurs thermiques. La concentration en donneurs thermiques est ensuite calculée à partir des valeurs de résistivité et d'un modèle mathématique exprimant la mobilité des porteurs de charge dans le silicium. On peut alors remonter à la concentration en oxygène interstitiel à partir de la concentration en donneurs thermiques.

Cette technique montre de bons résultats pour des échantillons en silicium cristallin faiblement dopé (dopage net $< 5.10^{16}$ cm$^{-3}$), notamment de type n. Par contre, lorsque les échantillons de silicium de type p sont fortement dopés (dopage net $\geq 5.10^{16}$ cm$^{-3}$), la génération des donneurs thermiques ne conduit pas à une variation de résistivité suffisante pour déterminer la concentration en oxygène interstitiel.

**Résumé de l'invention**

On constate qu'il existe un besoin de prévoir un procédé permettant de déterminer précisément la concentration en oxygène interstitiel dans tout type d'échantillon semi-conducteur de type p.

On tend à satisfaire ce besoin par les étapes suivantes :

- soumettre l'échantillon à un traitement thermique pour former des donneurs thermiques ;
- porter l'échantillon à une température comprise entre 0 K et 100 K et mesurer la concentration en porteurs de charge de l'échantillon ;
- déterminer la concentration en donneurs thermiques de l'échantillon à partir de la concentration en porteurs de charge et de la température de l'échantillon ; et
- déterminer la concentration en oxygène interstitiel à partir de la concentration en donneurs thermiques et de la durée du traitement thermique.

**Description sommaire des dessins**

D'autres avantages et caractéristiques ressortiront plus clairement de la description qui va suivre de modes particuliers de réalisation donnés à titre d'exemples non limitatifs et illustrés à l'aide des dessins annexés, dans lesquels :

- la figure 1 représente des étapes d'un procédé de détermination de la concentration en oxygène interstitiel $C_o$ selon l'invention ;
- la figure 2 représente un abaque de la concentration en porteurs de charge p dans un échantillon de silicium fortement dopé en fonction de la température T du recuit, pour différentes valeurs de la concentration en donneurs thermiques $N_{DDT}$ ;
- la figure 3 représente un abaque de la concentration en donneurs thermiques $N_{DDT}$ en fonction de la durée t du traitement thermique, pour différentes valeurs de la concentration en oxygène $C_o$ ; et
- la figure 4 représente des étapes supplémentaires du procédé de détermination de la concentration en oxygène interstitiel $C_o$ de la figure 1.

**Description d'un mode de réalisation préféré de l'invention**

Dans un substrat en silicium dopé de type p, les porteurs de charge majoritaires sont des trous. A température ambiante, leur concentration est définie par la concentration d'impuretés dopantes $N_A$ présentes dans le silicium, généralement des atomes de bore (B). Ces atomes sont appelés accepteurs d'électrons.

A l'inverse, dans un silicium de type n, les porteurs de charge majoritaires sont des électrons. Les impuretés dopantes sont des atomes donneurs d'électrons, par exemple des atomes de phosphore (P). A température ambiante, la concentration en électrons est donnée par la concentration en atomes donneurs $N_D$.

Par ailleurs, il existe des substrats dit « compensés » qui présentent les deux types d'impuretés dopantes, accepteurs et donneurs (en concentration $N_A$ et $N_D$ respectivement). Le silicium est alors de type p si $N_A$ est supérieur à $N_D$, et la concentration en trous vaut $N_A$-$N_D$ (à température ambiante). A l'inverse, le silicium est de type n si $N_D > N_A$, et la concentration en électrons vaut $N_D$-$N_A$ (à température ambiante).

En soumettant le substrat en silicium à une température comprise entre 200 °C et 500 °C, des donneurs thermiques DDT sont formés dans le substrat. Les donneurs thermiques sont considérés comme des impuretés dopantes de type donneur « double », car chaque DDT génère deux électrons.

L'activation des donneurs thermiques DDT provoque alors une variation de la concentration en porteurs de charge libres dans le substrat. Si celui-ci est de type n, la concentration d'électrons est augmentée de deux fois la concentration en donneurs thermiques $N_{DDT}$. Par contre, dans un substrat de type p, la concentration en trous est diminuée de deux fois la concentration $N_{DDT}$, suite à un rééquilibrage des charges.

On considère ci-après les semi-conducteurs de type p uniquement, et en exemple, le silicium.

Dans un silicium fortement dopé, la concentration en porteurs de charge libres, notée ci-après p, est élevée à température ambiante (p = $N_A$-$N_D \geq 5.10^{16}$ cm$^{-3}$). Ainsi, lorsqu'on veut mesurer la variation de la concentration p causée par l'activation des donneurs thermiques DDT, on constate que celle-ci est faible par rapport à la concentration initiale en porteurs de charge, de l'ordre de 4 à 5 décades. Il est alors très difficile de mesurer cette variation. De façon similaire, lorsque le silicium contient une faible teneur en oxygène interstitiel, les donneurs thermiques DDT activés sont peu nombreux. Dès lors, la concentration en porteurs de charge p n'est pratiquement pas modifiée lors du recuit.

Par contre, lorsqu'on observe cette variation à basse température, entre 0 K et 100 K, on constate que la contribution des donneurs thermiques sur la concentration en porteurs de charge p est significative. Cela vient du fait qu'à basse température, le nombre de porteurs de charge libres est considérablement réduit et devient du même ordre de grandeur que le nombre de donneurs thermiques DDT.

On propose ici de mettre en application ce constat pour déterminer de façon précise la concentration en oxygène interstitiel dans tout type de matériau semi-conducteur de type p, notamment dans un matériau fortement dopé et/ou contenant peu d'oxygène (à priori).

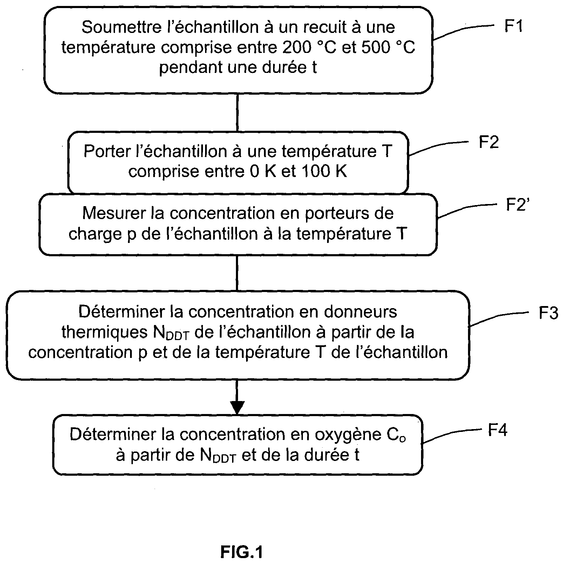

La figure 1 représente des étapes F1 à F4 d'un procédé de détermination de la concentration en oxygène interstitiel $C_o$.

Lors d'une première étape F1, un échantillon contenant de l'oxygène, par exemple un substrat en silicium, est soumis à un traitement thermique, ou recuit, pour former des donneurs thermiques DDT. La température du recuit est, de préférence, comprise entre 200 °C et 500 °C, et avantageusement entre 400 °C et 500 °C. En effet, comme cela sera décrit plus loin, la cinétique de formation des donneurs thermiques est bien connue dans cette plage de températures, notamment à 450 °C. La durée t du recuit est, de préférence, comprise entre 1 minute et 10 heures. Cette durée est fonction du niveau de dopage et de la teneur en oxygène du matériau : plus la teneur en oxygène est forte, plus la durée du recuit sera faible et plus la densité en porteurs de charge est élevée, plus la durée sera longue.

L'échantillon est ensuite porté, en F2, à une température T comprise entre 0 K et 100 K, et de préférence entre 4 K et 20 K. Puis, la concentration en porteurs de charge p de l'échantillon est mesurée à cette température (étape F2').

Plus la température T de mesure est basse, plus la précision sur la concentration en oxygène $C_0$ sera élevée. La plage 4-20 K est préférée car elle représente un bon compromis entre précision et facilité de mise en oeuvre. En effet, les températures de cette plage sont facilement atteignables, contrairement à des températures plus basses qui nécessitent des équipements plus complexes et onéreux.

La concentration en porteurs de charge p est, de préférence, mesurée par effet Hall. Cette technique est précise dans la plage de température susmentionnée, tout en étant simple à mettre en oeuvre. Toutefois, d'autres techniques pourront être envisagées, notamment celle basée sur une mesure de la capacité en fonction de la tension (C-V).

L'étape F3 du procédé de la figure 1 consiste à déterminer la concentration en donneurs thermiques $N_{DDT}$ de l'échantillon en silicium, à partir de la concentration en porteurs de charge p et de la température T de l'échantillon.

Pour cela, on a établi une relation p(T) liant la concentration en porteurs de charge p à la concentration en donneurs thermiques $N_{DDT}$ dans un échantillon de silicium, en fonction de la température T.

La relation p(T) est donnée ci-dessous dans le cas général d'un silicium compensé, c'est-à-dire comprenant des impuretés de type accepteur en concentration $N_A$, des impuretés de type donneur en concentration $N_D$ (autres que les donneurs DDT). Bien sûr, après le recuit de l'étape F1, le silicium comprend également des doubles donneurs thermiques en concentration $N_{DDT}$.

L'équation d'électroneutralité dans ce silicium s'écrit :

$$p = N_A^i - N_D - 2 \cdot N_{DDT} \qquad (1).$$

Le facteur 2 provient du caractère donneur « double » des DDT.

$N_A^i$ est la concentration en impuretés de type accepteur ionisées. Elle dépend de la concentration $N_A$ de la façon suivante (car toutes les impuretés de type accepteur ne sont pas ionisées à basse température) :

$$N_A^i = \frac{N_A}{1 + 4 \times \dfrac{p}{N_V \times e^{\frac{-E_{A_i}}{kT}}}} \qquad (2).$$

En remplaçant $N_A^i$ par son expression (2) dans la relation (1), puis en simplifiant, on obtient :

$$p^2 + p \times \left( \frac{N_V \times e^{\frac{-E_A}{kT}}}{4} + 2 \times N_{DDT} + N_D \right) + \left( N_D + 2 \times N_{DDT} - N_A \right) \left( \frac{N_V \times e^{\frac{-E_A}{kT}}}{4} \right) = 0$$

La solution de cette équation du second degré s'écrit :

$$p(T) = -\frac{1}{2}\left( 2 \times N_{DDT} + N_D + \frac{N_V}{4} e^{\frac{-E_A}{kT}} \right)$$
$$+ \frac{1}{2}\sqrt{ \left( 2 \times N_{DDT} + N_D + \frac{N_V}{4} e^{\frac{-E_A}{kT}} \right)^2 + \left( N_A - 2 \times N_{DDT} - N_D \right) \times N_V e^{\frac{-E_A}{kT}} } \qquad (3)$$

où $N_A$ est la concentration en impuretés dopantes de type accepteur, $N_D$ la concentration en impuretés dopantes de type donneur, $N_V$ la densité d'états équivalente dans la bande de valence, $E_A$ le niveau d'énergie des états accepteurs (calculé par rapport au haut de la bande de valence), k la constante de Boltzmann et T la température.

Les valeurs des paramètres $N_V$, $E_A$ et k, ainsi que leur variation éventuelle en fonction de la température T, sont connues de l'ouvrage « Physics of Semiconductor Devices » (S. M. Sze, Wiley-Interscience, New York, 1981).

Ainsi, lorsque le silicium de type p contient des impuretés dopantes de type accepteur seulement ($N_D = 0$), la relation p(T) s'écrit :

$$p(T) = -\frac{1}{2}\left( 2 \times N_{DDT} + \frac{N_V}{4} e^{\frac{-E_A}{kT}} \right)$$
$$+ \frac{1}{2}\sqrt{ \left( 2 \times N_{DDT} + \frac{N_V}{4} e^{\frac{-E_A}{kT}} \right)^2 + \left( N_A - 2 \times N_{DDT} \right) \times N_V e^{\frac{-E_A}{kT}} } \qquad (4).$$

A l'aide des relations (3) et (4) ci-dessus, on peut facilement calculer la concentration en donneurs thermiques $N_{DDT}$ à partir de la concentration en porteurs de charge p mesurée à une température T donnée.

La détermination de la concentration $N_{DDT}$ peut aussi être réalisée au moyen d'abaques.

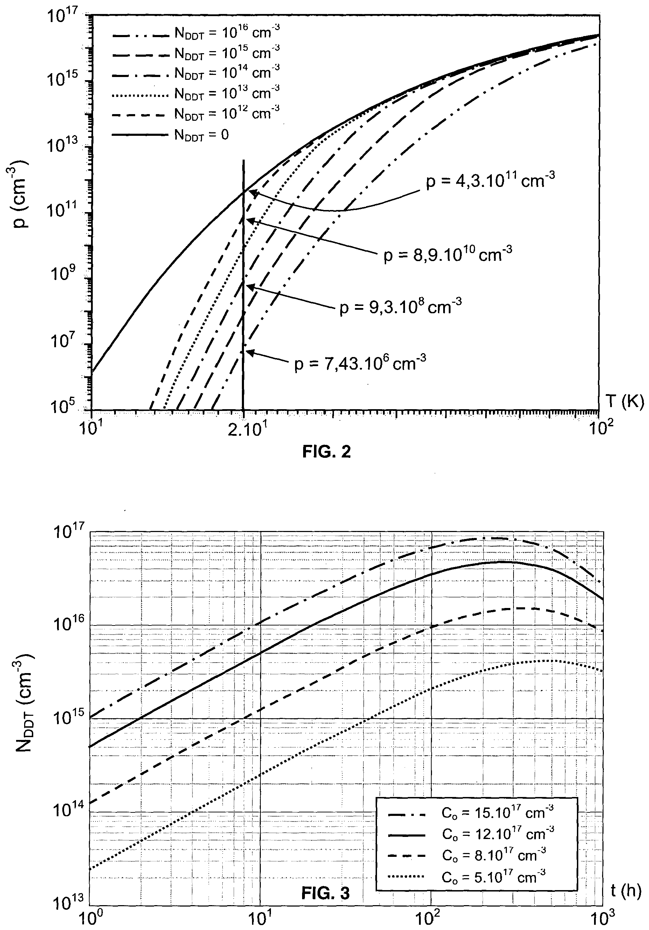

La figure 2 représente à titre d'exemple l'un de ces abaques. Celui-ci donne la concentration en porteurs de charge p en fonction de la température T, pour différentes valeurs de $N_{DDT}$. Il a été établi à partir de la relation (4) pour un substrat en silicium de type p, dopé au bore ($N_A = 10^{17}$ cm$^{-3}$).

A titre de comparaison, on a également représenté sur l'abaque de la figure 2 la concentration en trous p(T) pour un même substrat, mais dépourvu de donneurs thermiques (courbe en trait plein).

On remarque que la formation de donneurs thermiques DDT entraîne des réductions significatives de la concentration en trous p. A 20 K par exemple, la concentration p vaut $4,3.10^{11}$ cm$^{-3}$ en l'absence de donneurs thermiques. Elle chute à $8,9.10^{10}$ cm$^{-3}$ pour une concentration $N_{DDT}$ égale à $10^{12}$ cm$^{-3}$, à $9,3.10^{8}$ cm$^{-3}$ pour une concentration $N_{DDT}$ de $10^{14}$ cm$^{-3}$ et à $7,43.10^{6}$ cm$^{-3}$ pour une concentration $N_{DDT}$ de $10^{16}$ cm$^{-3}$. La concentration p dépend donc fortement de la concentration $N_{DDT}$ dans la plage de températures 0 K-100 K.

La lecture de l'abaque, pour une concentration p et une température T données, permet d'obtenir la concentration en donneurs thermiques $N_{DDT}$.

A l'étape F4 (Fig.1), on détermine la concentration en oxygène interstitiel $C_O$ de l'échantillon à partir de la durée t du recuit et de la concentration en donneurs thermiques $N_{DDT}$ calculée à l'étape F3.

La concentration en oxygène interstitiel $C_O$ est, de préférence, calculée à l'aide d'une relation tirée de l'article « Formation kinetics of oxygen thermal donors in silicon » (Wijaranakula C.A. et al., Appl. Phys. Lett. 59 (13), pp. 1608, 1991). Cet article décrit la cinétique de formation des donneurs thermiques dans le silicium par un recuit à 450 °C.

Cette température constitue d'ailleurs un bon compromis entre la vitesse de formation des donneurs thermiques et la concentration maximale obtenue. Une température supérieure à 450 °C favorise la vitesse de formation des DDT au détriment de la concentration maximale. Une température élevée est donc à privilégier lorsque l'on suppose que la concentration en oxygène est importante, par exemple supérieure à $5.10^{17}$ cm$^{-3}$. A l'inverse, une température inférieure à 450 °C permettra d'augmenter la concentration maximale de DDT et pourra être utilisée pour des substrats dont la concentration approximative en oxygène est faible, par exemple inférieure à $5.10^{17}$ cm$^{-3}$.

Sans informations préalables sur la concentration en oxygène, on choisira, de préférence, une température de recuit comprise entre 400 °C et 500 °C, par exemple égale à 450 °C.

La relation exprimant la concentration en donneurs thermiques $N_{DDT}$ en fonction de la concentration en oxygène $C_O$ et de la durée de recuit t est donnée ci-dessous :

$$N_{DDT}(t,C_o) = 4,51.10^{-52} \times \left( C_o \left[ 1 + \frac{2}{3} D_o \times t \times C_o^{2/3} \right]^{-3/2} \right)^{3,45} \times t^{1,02} \qquad (5),$$

avec $D_o$ le coefficient de diffusion de l'oxygène interstitiel $\left( D_o = 0,13 \times e^{-\frac{2,53}{kT}} \right)$.

Connaissant t et $N_{DDT}$, on peut calculer facilement la concentration en oxygène interstitiel $C_O$ du substrat.

Alternativement, la concentration en oxygène interstitiel $C_O$ peut être déterminée à l'aide d'abaques de la concentration en donneurs thermiques $N_{DDT}$ en fonction de la durée de recuit t, pour différentes valeurs de la concentration en oxygène $C_O$.

La figure 3 représente l'un de ces abaques, construit à partir de la relation (5) et pour une température de recuit de l'ordre de 450 °C. On peut remarquer qu'une faible variation de la concentration en oxygène $C_O$ conduit à une forte variation de la concentration en donneurs thermiques $N_{DDT}$.

La lecture de cet abaque permet de déterminer la valeur de la concentration $C_O$, pour une concentration $N_{DDT}$ et une durée de recuit t données.

Pour une température de recuit différente de 450 °C, la relation (5) et les abaques peuvent être adaptés, notamment grâce aux enseignements de l'article « Effect of oxygen concentration on the kinetics of thermal donor formation in silicon at temperatures between 350 and 500 °C » (Londos C.A. et al., Appl. Phys. Lett. 62 (13), pp. 1525, 1993). Cet article décrit également la cinétique de formation des donneurs thermiques dans le silicium, mais pour des températures de recuit comprises entre 350 °C et 500 °C.

Ainsi, en combinant une mesure de p à basse température et un modèle mathématique p(T) qui tient compte des donneurs thermiques, il est possible de déterminer précisément la concentration $N_{DDT}$ et d'en déduire la concentration en oxygène interstitiel.

A titre d'exemple, si la concentration p est mesurée à 10 K, la limite de détection de $C_0$ est abaissée à $10^{15}$ cm$^{-3}$. En d'autres termes, il est possible de mesurer des concentrations en oxygène aussi faibles que $10^{15}$ cm$^{-3}$, ce que ne permettent pas les techniques de l'art antérieur.

Le calcul de $N_{DDT}$ réalisé à l'étape F3 nécessite de connaître la valeur de la concentration en impuretés dopantes de type accepteur $N_A$, et éventuellement la concentration en impuretés dopantes de type donneur $N_D$ (type p compensé ou non). Ces valeurs peuvent être données par le fournisseur des substrats. Dans le cas contraire, elles peuvent être déterminées lors d'une étape supplémentaire du procédé de la figure 1.

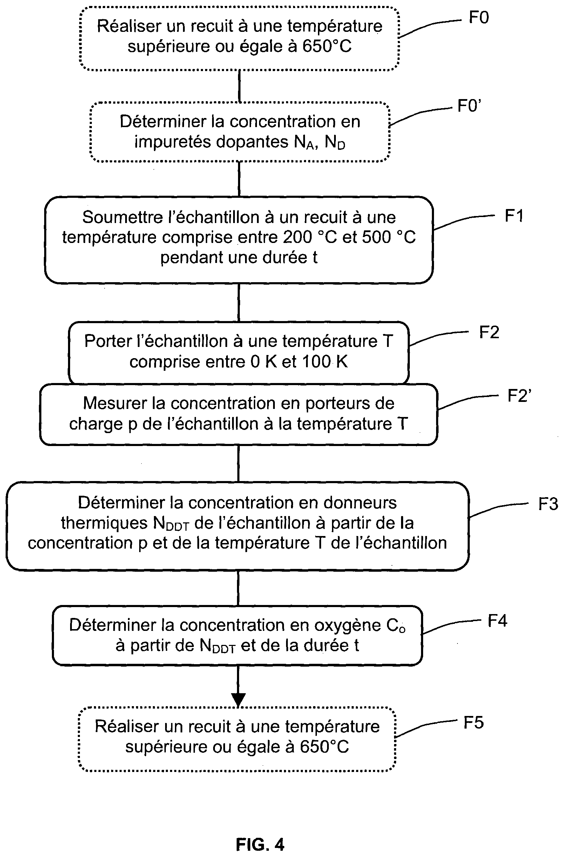

La figure 4 représente des étapes supplémentaires du procédé de détermination, dont une permet de déterminer la concentration en impuretés dopantes $N_A$ ou $N_D$.

De préférence, la concentration $N_A$ ou $N_D$ est déterminée lors d'une étape F0' préalablement à l'étape de recuit F1.

Pour du silicium de type p non compensé (relation 4), la concentration $N_A$ peut être calculée à l'aide de la relation suivante, après avoir mesuré la résistivité initiale $\rho_0$ du substrat :

$$\rho_0 = \frac{1}{N_A \cdot q \cdot \mu_p} \qquad (6) \; ;$$

q étant la charge élémentaire (q = 1,6.10$^{-19}$ C) et $\mu_p$ la mobilité des trous dans le silicium.

La mesure de résistivité peut être réalisée de façon simple par la méthode des quatre pointes ou par une méthode sans contact, par exemple par couplage inductif.

Afin de s'assurer que le substrat ne comporte pas de donneurs thermiques dans son état initial, ce qui pourrait fausser la valeur de $N_A$, on procède, de préférence, à un recuit, en F0, à une température supérieure ou égale à 650 °C. Cela rend les précipités d'oxygène (ou donneurs thermiques DDT) instables et les élimine. Les atomes d'oxygène reprennent alors leurs positions interstitielles. La mesure de $\rho_0$ est donc avantageusement réalisée après un tel recuit.

Le recuit F0 peut d'ailleurs être réalisé même lorsque la concentration $N_A$ est connue, afin de s'assurer que la concentration en donneurs thermiques $N_{DDT}$ est initialement nulle. Autrement dit, l'étape F0 peut être réalisée en l'absence de l'étape F0'.

Un tel recuit est, de préférence, également utilisé à la fin du procédé, en F5, après avoir déterminé la concentration en oxygène interstitiel (F4). Grâce à cette étape de recuit F5, le substrat revient dans son état initial et peut être réutilisé.

Dans le cas du silicium compensé (relation 3), il est nécessaire de connaître à la fois la valeur de $N_A$ et la valeur de $N_D$. On pourra, comme précédemment, mesurer la résistivité initiale du substrat, à condition de connaître l'une de ces deux valeurs.

Si aucune d'entre elles n'est connue, on pourra faire des mesures supplémentaires, notamment une mesure de type GDMS (Glow Discharge Mass Spectroscopy).

Dans un mode de réalisation préférentiel (non représenté), la concentration en porteurs de charge p est mesurée pour différentes températures T comprises entre 0 K et 100 K. On obtient alors plusieurs couples de valeurs {p, T} que l'on peut corréler à la relation (3) ou (4) pour déterminer la concentration $N_{DDT}$. Il n'est alors même plus nécessaire de connaître la concentration en impuretés dopantes $N_A$ dans le cas de la relation (4). La connaissance de $N_D$ reste par contre nécessaire dans le cas du silicium compensé (relation 3).

De nombreuses variantes et modifications du procédé de détermination décrit ici apparaîtront à l'homme du métier. Le procédé a été décrit en relation avec un substrat de silicium. Toutefois, le procédé peut être également appliqué aux autres semi-conducteurs du groupe IV dopé de type p, notamment des substrats de germanium ou de silicium-germanium. En effet, le germanium est également un semi-conducteur dans lequel des donneurs thermiques peuvent être formés en présence d'oxygène.

**Revendications**

1.  Procédé de détermination de la concentration en oxygène interstitiel ($C_O$) d'un échantillon en matériau semi-conducteur de type p, comportant les étapes suivantes :

    a) soumettre (F1) l'échantillon à un traitement thermique pour former des donneurs thermiques (DDT) ;
    b) porter (F2) l'échantillon à une température (T) comprise entre 0 K et 100 K et mesurer (F2') la concentration en porteurs de charge (p) de l'échantillon ;
    c) déterminer (F3) la concentration en donneurs thermiques ($N_{DDT}$) de l'échantillon à partir de la concentration en porteurs de charge (p) et de la température (T) de l'échantillon; et
    d) déterminer la concentration en oxygène interstitiel à partir de la concentration en donneurs thermiques et de la durée (t) du traitement thermique.

2.  Procédé selon la revendication 1, comprenant une pluralité de mesures de la concentration en porteurs de charge (p) à différentes températures comprises entre 0 K et 100 K, et dans lequel la concentration en donneurs thermiques ($N_{DDT}$) est déterminée par corrélation des mesures avec une relation (p(T)) décrivant l'évolution de la concentration en porteurs de charge en fonction de la température.

3.  Procédé selon l'une des revendications 1 et 2, dans lequel, l'échantillon comprenant des impuretés dopantes de type accepteur et donneur, la concentration en donneurs thermiques $N_{DDT}$ est déterminée à l'aide de la relation suivante :

# EP 2 834 625 B1

$$p(T) = -\frac{1}{2}\left(2 \times N_{DDT} + N_D + \frac{N_V}{4} e^{\frac{-E_A}{kT}}\right)$$

$$+ \frac{1}{2}\sqrt{\left(2 \times N_{DDT} + N_D + \frac{N_V}{4} e^{\frac{-E_A}{kT}}\right)^2 + \left(N_A - 2 \times N_{DDT} - N_D\right) \times N_V e^{\frac{-E_A}{kT}}}$$

dans laquelle $N_A$ est la concentration en impuretés dopantes de type accepteur, $N_D$ est la concentration en impuretés dopantes de type donneur, $E_A$ le niveau d'énergie des états accepteurs, $N_V$ la densité d'états équivalente dans la bande de valence, k la constante de Boltzmann et T la température.

4.  Procédé selon la revendication 3, comprenant, avant l'étape (F1) de traitement thermique, une étape (F0') de détermination de la concentration en impuretés dopantes ($N_A$, $N_D$) à partir d'une mesure de résistivité de l'échantillon.

5.  Procédé selon l'une quelconque des revendications 1 à 4, comprenant initialement une étape (F0) de recuit à une température supérieure ou égale à 650°C.

6.  Procédé selon l'une quelconque des revendications 1 à 5, dans lequel la concentration en porteurs de charge (p) est mesurée par effet Hall.

7.  Procédé selon l'une quelconque des revendications 1 à 6, dans lequel le traitement thermique est réalisé à une température comprise entre 200 °C et 500 °C.

8.  Procédé selon la revendication 7, dans lequel la température du traitement thermique est comprise entre 400 °C et 500 °C.

9.  Procédé selon l'une quelconque des revendications 1 à 8, dans lequel l'échantillon est porté (F2) à une température comprise entre 4 K et 20 K pour mesurer (F2') la concentration en porteurs de charge (p).


**Patentansprüche**

1.  Verfahren zur Bestimmung der Konzentration von interstitiellem Sauerstoff ($C_O$) in einem Probestück aus p-Halbleitermaterial, das folgende Verfahrensschritte umfasst:

    a) das Probestück wird einer Wärmebehandlung unterzogen (F1), um thermische Donatoren (DDT) zu bilden;,
    b) das Probestück wird auf eine Temperatur (T) gebracht (F2), die zwischen 0 K und 100 K beträgt, und die Konzentration von Ladungsträgern (p) des Probestücks wird gemessen (F2'),
    c) Bestimmung (F3) der Konzentration von thermischen Donatoren ($N_{DDT}$) in dem Probestück aus der Konzentration von Ladungsträgern (p) und der Temperatur (T) des Probestücks; und
    d) Bestimmen der Konzentration von interstitiellem Sauerstoff aus der Konzentration von thermischen Donatoren und der Dauer (t) der Wärmebehandlung.

2.  Verfahren nach Anspruch 1, welches eine Mehrzahl von Messungen der Konzentration von Ladungsträgern (p) bei verschiedenen Temperaturen von 0 K bis 100 K umfasst, und bei dem die Konzentration von thermischen Donatoren ($N_{DDT}$) durch Korrelation der Messungen mit einer Relation (p(T)), die die Veränderung der Konzentration von Ladungsträgern in Abhängigkeit von der Temperatur beschreibt, bestimmt wird.

3.  Verfahren nach einem der Ansprüche 1 und 2, bei dem an dem Probestück, das Dotierstoffe des Typs Akzeptor- und Donator-Fremdstoffe enthält, die Konzentration von thermischen Donatoren ($N_{DDT}$) mittels der folgenden Relation bestimmt wird:

$$p(T) = -\frac{1}{2}\left(2 \times N_{DDT} + N_D + \frac{N_V}{4}e^{\frac{-E_A}{kT}}\right)$$
$$+\frac{1}{2}\sqrt{\left(2 \times N_{DDT} + N_D + \frac{N_V}{4}e^{\frac{-E_A}{kT}}\right)^2 + (N_A - 2 \times N_{DDT} - N_D) \times N_V e^{\frac{-E_A}{kT}}}$$

wobei $N_A$ die Konzentration von Dotierstoffen des Typs Akzeptor-Fremdstoffe ist, $N_D$ die Konzentration von Dotierstoffen des Typs Donator-Fremdstoffe ist, $E_A$ das Energieniveau der Akzeptorzustände ist, $N_V$ die gleichwertige Zustandsdichte im Valenzband ist, k die Boltzmannkonstante und T die Temperatur ist.

4. Verfahren nach Anspruch 3, welches vor dem Verfahrensschritt (F1) der Wärmebehandlung einen Verfahrensschritt (F0') der Bestimmung der Konzentration von Dotierstoffen ($N_A$, $N_D$) auf Grundlage einer Messung des spezifischen Widerstandes des Probestücks enthält.

5. Verfahren nach einem der Ansprüche 1 bis 4, welches zu Beginn einen Verfahrensschritt (F0) des Glühens bei einer Temperatur von 650°C oder darüber enthält.

6. Verfahren nach einem der Ansprüche 1 bis 5, bei dem die Konzentration von Ladungsträgern (p) durch Halleffekt gemessen wird.

7. Verfahren nach einem der Ansprüche 1 bis 6, bei dem die Wärmebehandlung bei einer Temperatur von 200°C bis 500°C durchgeführt wird.

8. Verfahren nach Anspruch 7, bei dem die Temperatur der Wärmebehandlung zwischen 400°C und 500°C beträgt.

9. Verfahren nach einem der Ansprüche 1 bis 8, bei dem das Probestück auf eine Temperatur, die zwischen 4 K und 20 K beträgt, gebracht wird (F2), um die Konzentration von Ladungsträgern (p) zu messen (F2').

**Claims**

1. A method for determining the interstitial oxygen concentration ($C_o$) of a sample made from p-type semiconductor material comprising the following step:

   a) subjecting (F1) the sample to thermal treatment to form thermal donors (TDD);
   b) bringing (F2) the sample to a temperature (T) comprised between 0 K and 100 K and measuring (F2') the charge carrier concentration (p) of the sample;
   c) determining (F3) the thermal donor concentration ($N_{TDD}$) of the sample from the charge carrier concentration (p) and the temperature (T) of the sample; and
   d) determining the interstitial oxygen concentration ($C_o$) from the thermal donor concentration and from duration of the thermal treatment.

2. The method according to claim 1, comprising a plurality of measurements of the charge carrier concentration (p) at different temperatures comprised between 0 K and 100 K, and wherein the thermal donor concentration ($N_{TDD}$) is determined by correlation of the measurements with a relation (p(T)) describing the variation of the charge carrier concentration versus the temperature.

3. The method according to one of claims 1 and 2, wherein, the sample comprising dopant impurities of acceptor and donor type, the thermal donor concentration $N_{TDD}$ is determined by means of the following relation:

$$p(T) = -\frac{1}{2}\left(2 \times N_{TDD} + N_D + \frac{N_V}{4} e^{\frac{-E_A}{kT}}\right)$$

$$+\frac{1}{2}\sqrt{\left(2 \times N_{TDD} + N_D + \frac{N_V}{4} e^{\frac{-E_A}{kT}}\right)^2 + \left(N_A - 2 \times N_{TDD} - N_D\right) \times N_V e^{\frac{-E_A}{kT}}}$$

wherein $N_A$ is the concentration of dopant impurities of acceptor type, $N_D$ is the concentration of dopant impurities of donor type, $E_A$ the energy level of the acceptor states, $N_V$ the equivalent density of states in the valence band, k the Boltzmann's constant and T the temperature.

4. The method according to claim 3, comprising, before the thermal treatment step (F1), a step (F0') of determining the dopant impurity concentration ($N_A$, $N_D$) from a resistivity measurement of the sample.

5. The method according to any one of claims 1 to 4, initially comprising an annealing step (F0) at a temperature greater than or equal to 650°C.

6. The method according to any one of claims 1 to 5, wherein the charge carrier concentration (p) is measured by Hall effect.

7. The method according to any one of claims 1 to 6, wherein the thermal treatment is performed at a temperature comprised between 200 °C and 500 °C.

8. The method according to claim 7, wherein the temperature of the thermal treatment is comprised between 400 °C and 500 °C.

9. The method according to any one of claims 1 to 8, wherein the sample is brought (F2) to a temperature comprised between 4 K and 20 K to measure (F2') the charge carrier concentration (p).

Soumettre l'échantillon à un recuit à une
température comprise entre 200 °C et 500 °C
pendant une durée t — F1

Porter l'échantillon à une température T
comprise entre 0 K et 100 K — F2

Mesurer la concentration en porteurs de
charge p de l'échantillon à la température T — F2'

Déterminer la concentration en donneurs
thermiques $N_{DDT}$ de l'échantillon à partir de la
concentration p et de la température T de l'échantillon — F3

Déterminer la concentration en oxygène $C_o$
à partir de $N_{DDT}$ et de la durée t — F4

FIG.1

**FIG. 2**

**FIG. 3**

Réaliser un recuit à une température
supérieure ou égale à 650°C — F0

Déterminer la concentration en
impuretés dopantes $N_A$, $N_D$ — F0'

Soumettre l'échantillon à un recuit à une
température comprise entre 200 °C et 500 °C
pendant une durée t — F1

Porter l'échantillon à une température T
comprise entre 0 K et 100 K — F2

Mesurer la concentration en porteurs de
charge p de l'échantillon à la température T — F2'

Déterminer la concentration en donneurs
thermiques $N_{DDT}$ de l'échantillon à partir de la
concentration p et de la température T de l'échantillon — F3

Déterminer la concentration en oxygène $C_o$
à partir de $N_{DDT}$ et de la durée t — F4

Réaliser un recuit à une température
supérieure ou égale à 650°C — F5

**FIG. 4**

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2964459

**Littérature non-brevet citée dans la description**

- **A.G. ULYASHIN et al.** Characterization of the oxygen distribution in Czochralski silicon using hydrogen-enhanced thermal donor formation. *Materials Science and Engineering B73,* 2000, 124-129
- **E. SIMOEN et al.** Characterisation of oxygen and oxygen-related defects in highly-and lowly-doped silicon. *Materials Science and Engineering B102,* 2003, 207-212
- **S. M. SZE.** Physics of Semiconductor Devices. Wiley-Interscience, 1981
- **WIJARANAKULA C.A. et al.** Formation kinetics of oxygen thermal donors in silicon. *Appl. Phys. Lett.,* 1991, vol. 59 (13), 1608
- **LONDOS C.A. et al.** Effect of oxygen concentration on the kinetics of thermal donor formation in silicon at temperatures between 350 and 500 °C. *Appl. Phys. Lett.,* 1993, vol. 62 (13), 1525